# EUROPÄISCHE PATENTSCHRIFT

(11) **EP 1 145 739 B1**
(45) Veröffentlichungstag und Bekanntmachung des Hinweises auf die Patenterteilung: **15.09.2010**
(21) Anmeldenummer: 01116136.1
(22) Anmeldetag: 11.07.1996
(51) Int. Cl.: A61N 5/10

(54) **Bestrahlungsgerät**
Irradiation unit
Appareil d'exposition à des rayonnements

(30) Priorität: 15.07.1995 DE 19525811; 24.05.1996 DE 29609310 U
(43) Veröffentlichungstag der Anmeldung: 17.10.2001
(62) Teilanmeldung aus: 96925727.8
(73) Patentinhaber: Varian Medical Systems Haan GmbH, 42781 Haan (DE)
(72) Erfinder: Schreckenberg, Wolfgang, 40468 Düsseldorf (DE); Kindlein, Johann, 46049 Oberhausen (DE); Rohe, Karl-Hienz, 42781 Haan (DE); Weinlich, Karl, 42289 Wuppertal (DE)
(74) Vertreter: Kühn, Armin

(56) Entgegenhaltungen:
- EP-A- 0 138 088
- EP-A- 0 278 829
- DE-A- 3 643 902

## Beschreibung

Die Erfindung betrifft ein Bestrahlungsgerät mit zumindest einer mittels eines Transportkabels zwischen zumindest einer Ruheposition und zumindest einer Bestrahlungsposition bewegbaren, auswechselbaren Strahlenquelle und mit einem Transportbehälter für den Transport auszuwechselnder Strahlenquellen.

Ein derartiges Bestrahlungsgerät ist zum Beispiel aus der EP-B1-0 138 088 bekannt, deren Offenbarungsgehalt durch diesen Verweis in den Offenbarungsgehalt vorliegender Beschreibung aufgenommen wird.

Erhöhte Sicherheitsanforderungen werden an gattungsgemäße Bestrahlungsgeräte gestellt, wenn diese zur intraarteriellen Bestrahlung zur Verhinderung einer postangioplastischen Restenosis Verwendung finden sollen. Ein derartiges Bestrahlungsgerät und Behandlungsverfahren ist u. a. in der EP-A1-0 633 041 beschrieben worden. Diese Druckschrift wird durch Bezugnahme auf sie zum Offenbarungsgehalt der vorliegenden Patentanmeldung gemacht.

Insbesondere beim Strahlenquellenwechsel unterliegen die bekannten Bestrahlungsgeräte einem gewissen Fehlbedienungsrisiko, so daß ein Strahlenquellenwechsel nur durch besonders geschultes Personal durchgeführt werden kann. Hierbei werden dem Anwender die Strahlenquellen in einem, in der Regel zweikanalig ausgeführten Transportbehälter angeliefert. Der eine Kanal dient zur Aufnahme der alten, d. h. der auszutauschenden Strahlenquellen, während der andere Kanal zur Aufnahme der frischen Strahlenquelle dient. Das Bestrahlungsgerät wird über einen Transportschlauch mit dem leeren Kanal des Transportbehälters verbunden. Anschließend wird die auszutauschende Strahlenquelle durch das Transportkabel in den Transportbehälter gefahren. Das antriebseitige Transportkabelende wird vom Antrieb gelöst, nachdem das strahlerseitige Ende im Transportbehälter gegen Verrutschen festgelegt worden ist. Anschließend wird der Transportschlauch mit dem die frische Strahlenquelle enthaltenden Kanal verbunden und die frische Strahlenquelle in umgekehrter Reihenfolge zur alten Strahlenquelle in das Bestrahlungsgerät überführt. Der Strahlenquellenwechsel ist eine kritische Situation, da die Strahlenquelle dafür zwingend die Abschirmung des Bestrahlungsgerätes über den Transportschlauch verlassen muß und da das Transportkabel völlig gelöst bzw. neu angekoppelt werden muß. Insbesondere kann jede beliebige Strahlenquelle in das Bestrahlungsgerät eingezogen werden, so daß es über den Verbleib alter bzw. neuer Strahlenquellen zu Zweifeln oder Irrtümern kommen kann.

Der Erfindung liegt somit die Aufgabe zugrunde, ein Bestrahlungsgerät bereit zu stellen, bei welchem das Risiko einer Fehlbedienung reduziert ist.

Als Lösung wird ein Bestrahlungsgerät mit den Merkmalen der Ansprüche 1, 3 und/oder 4 vorgeschlagen.

Bei erfindungsgemäßen Bestrahlungsgeräten ist die Strahlungsquelle in ihrer Nichtgebrauchsstellung beim Anwender (Ruheposition) in einem Abschirmblock gelagert und wird - wie üblich - mittels eines Transportkabels in die Bestrahlungsposition bewegt. Die Abschirmung umgibt die Strahlenquelle derart, daß die Durchlaßstrahlung des Bestrahlungsgerätes dann den für Strahlenquellenlagerbehälter geltenden Bestimmungen für die Lagerung und für die Anwendung von Strahlenquellen entspricht. Erfindungsgemäß dient der für den Fern-Transport der Strahlenquelle zwischen dem Lieferanten und dem Anwender (Kunde) verwendete Strahlenschutzbehälter (Transportbehälter) als integrierter bzw. integrierbarer Bestandteil des Bestrahlungsgerätes. Er wird also wie ein Schlüssel (Transportbehälter) in ein Schloß (übriger Teil des Bestrahlungsgerätes) eingefügt, wofür entsprechende Schnittstellenmittel vorgesehen sind. - Erst dadurch entsteht eine verwendbare Einheit (Bestrahlungsgerät) (Anspruch 1). Hierdurch wird vermieden, daß die Strahlenquelle während des Strahlenquellenaustausches gegen zum Beispiel eine frische Strahlenquelle ihre Abschirmung noch beim Anwender (Kunden) verlassen muß. Des weiteren kann hierdurch vermieden werden, daß es über den Verbleib gebrauchter bzw. frischer Strahlenquellen zu Zweifeln kommen kann, da jeweils eine Strahlenquelle genau einem Fern-Transportbehälter zugeordnet ist.

Diese Geräteeigenschaften bringen dem Anwender insbesondere den Vorteil, problemlos mit Quellen unterschiedlicher Spezifikation (hinsichtlich Aktivität, Energie und Geometrie) arbeiten zu können. So ist ein Wechsel von HDR- zu PDR- (Puls-Dose- Rate) Betrieb und der Einsatz einer dem Anwendungsfall optimal angepassten Quelle leicht möglich.

Um das Transportgewicht eines erfindungsgemäßen Transportbehälters nicht unverhältnismäßig erhöhen zu müssen, kann der Transportbehälter eine den für Strahlenquellenferntransportbehälter geltenden Bestimmungen entsprechende Abschirmungen umfassen und der übrige Teil des Bestrahlungsgerätes eine Abschirmung aufweisen, die zumindest Teile des Transportbehälters derart umgibt, daß die Durchlaßstrahlung des Bestrahlungsgerätes bezüglich seiner Ruheposition den für Strahlenquellenlagerbehälter geltenden (strengeren) Bestimmungen entspricht (Anspruch 2). Hierbei versteht es sich, daß die Abschirmung des Fern-Transportbehälters in bestimmten Raumrichtungen bereits den Bestimmungen für Strahlenquellenlagerbehälter entsprechen kann, während sie in anderen Raumrichtungen hierfür noch nicht ausreichend stark ist. Hierbei gilt es einen Kompromiß zwischen Bedienungskomfort beim Einbringen des Transportbehälters in den Bestrahlungsgerät und Transportgewicht zu finden.

Der Aufbau eines erfindungsgemäßen Bestrahlungsgerätes läßt sich vereinfachen, wenn der Transportbehälter einen durchgehenden spiralenförmigen Transportkanal aufweist, innerhalb dessen sich die Lagerposition befindet. An einem Ende des Tranportkanals kann das Transportkabel eintreten und mit einem vorgeschalteten Antrieb, wie zum Beispiel einer Speichertrommel, verbunden sein. Um die Strahlenquelle aus ihrer Ruheposition in ihre Bestrahlungsposition zu überführen, schiebt das Transportkabel die Strahlenquelle vor sich her durch den Transportkanal.

Die den Transportbehälter in Betriebstellung umgebende Abschirmung des Bestrahlungsgerätes kann eine, vorzugsweise aufklappbare, Abschirmungshülle umfassen. Hierdurch wird der Strahlenquellenwechsel vereinfacht und sichergestellt, daß die Gesamt-Abschirmung nach Einbringen des Transportbehälters in das Bestrahlungsgerät den für Strahlenquellenlagerbehälter geltenden Bestimmungen entspricht.

Zur weiteren Vermeidung einer Fehlbedienung können Mittel zum automatischen Blockieren der Strahlenquelle bzw. des Transportkabels in einer Transportposition beim Transport des Transportbehälters vorgesehen sein. Hierdurch kann ein Vergessen des Blockierens der Strahlenquelle vor dem Transport vermieden werden. Vorzugsweise werden die Strahlenquelle bzw. das Transportkabel automatisch blockiert, wenn der Transportbehälter von dem Bestrahlungsgerät entfernt wird.

An dem Transportbehälter kann ein Transportkabelspeicher, insbesondere eine Speichertrommel vorgesehen sein, auf bzw. von welcher das Transportkabel auf- bzw. abgewickelt wird, so daß Transportbehälter und ein Transportkabelspeicher, insbesondere Speichertrommel, zu einem Transportmodul zusammengefaßt sind (Anspruch 3). Hierdurch wird vermieden, daß das Transportkabel zum Transport der Strahlenquelle von seiner Antriebseinheit getrennt werden muß, ein Vorgang, bei welchem die verschiedensten, insbesondere Bedienungs-Fehler auftreten könnten. Es versteht sich, daß das Transportmodul weitere Transportkabelantriebsmittel umfassen kann. Es kann jedoch - insbesondere aus Gewichtsgründen - von Vorteil sein, Transportkabelantriebsmittel - wie den Kraftantrieb - in dem übrigen Teil des Bestrahlungsgerätes vorzusehen, die durch geeignete Kupplungen mit dem Transportmodul bzw. dem Transportkabelspeicher verbunden werden, wenn das Transportmodul in das übrige Bestrahlungsgerät angebracht wird. Hierbei versteht es sich, daß diese Kopplung, vorzugsweise automatisch erfolgen kann. - Die modulartige Zusammenfassung von Transportbehälter und Transportkabelspeicher hat ferner den Vorteil erhöhter Präzision bei der Positionsbestimmung des Strahlers, da z. B. etwaige Fertigungstoleranzen als unveränderliche Korrekturgröße berücksichtigt werden können.

Zur Erhöhung der Sicherheit, kann das Transportmodul Mittel zum, vorzugsweise automatischen, Blockieren der Speichertrommel beim Transport des Transportmoduls umfassen. Hierdurch wird eine Betätigung des Transportkabelantriebs während des Strahlenquellentransportes verhindert.

Vorzugsweise werden die Blockiermittel vor Einleitung der Bewegung der Strahlenquelle aus ihrer Ruheposition in die Bestrahlungsposition automatisch freigegeben (Anspruch 5). Hierdurch können Beschädigungen an den Baugruppen des erfindungsgemäßen Bestrahlungsgerätes einschließlich des Transportbehälters bzw. des Transportmoduls vermieden werden. Vorzugsweise werden die Blockiermittel automatisch beim Einbau des Transportmoduls bzw. -behälters an das Bestrahlungsgerät freigegeben.

Insbesondere bei Bestrahlungen in Herznähe werden extrem dünne Transportkabel mit Gamma- oder Beta- Strahlenquellen benötigt.

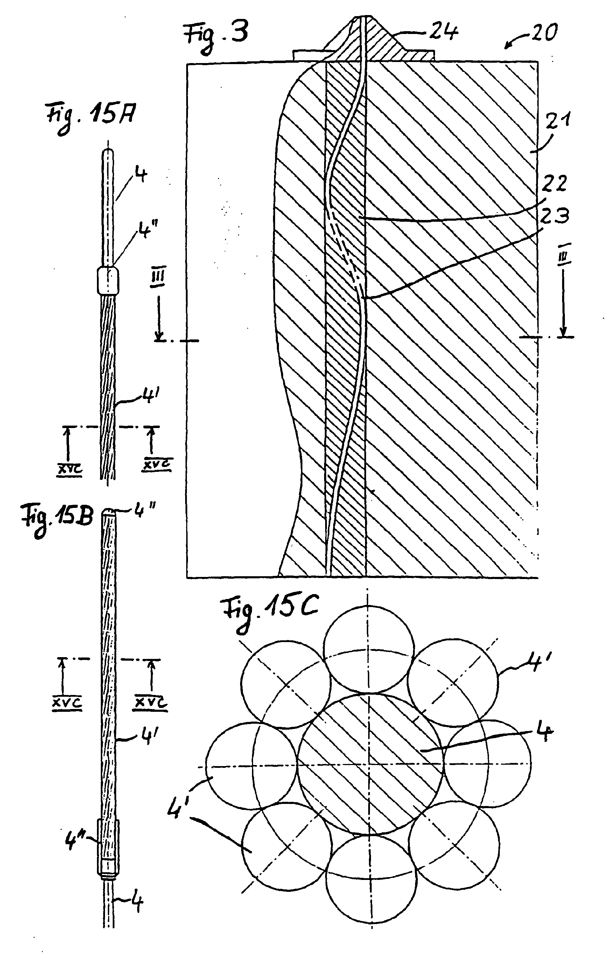

Um Strahlenquellen durch Katheter mit extrem kleinen Biegeradien schieben zu können, sind flexible, mit einem hochflexiblen Vorläuferdraht versehene Quellen von Vorteil (s. Fig. 15).

Um z. B. ein Ausknicken des Transportkabels zu vermeiden, wird ein Transportkabelantrieb vorgeschlagen, mit dem auch extrem dünne Transportkabel knick- und schlaufenfrei angetrieben werden können. Hierzu wird das Transportkabel auf bzw. von einer ansich bekannten Speichertrommel mit einer, zumindest teilweise durch ein Anpreßband abgedeckten Kabelaufnahmenut auf- bzw. abgewickelt, wobei ein neuartiges Anpreßband an dem auf der Speichertrommel spiralförmig aufgewickelten Teil des Transportkabels weitgehend parallel zu dem Transportkabel auf der Speichertrommel aufgewickelt ist (Anspruch 4). Das Anpreßband ist vergleichsweise schmal und deckt in seiner Breitenerstreckung nur einen Gang des Transportkabels ab. Dabei kann das Transportkabel in axialer oder in radialer Richtung spiralig auf die Speichertrommel aufgewickelt werden. Folglich preßt das Anpreßband das Transportkabel bis auf einen sehr kleinen Bereich unmittelbar vor dem Abhebepunkt des Transportkabels von der Speichertrommel in die Kabelaufnahmenut. Im Gegensatz zu den aus dem Stand der Technik bekannten Anpreßbändern wird in diesem Falle das Transportkabel praktisch ohne nennenswerte Unterbrechung über seine gesamte auf der Speichertrommel aufgewickelte Länge an die Speichertrommel bzw. in die Kabelaufnahmenut gepreßt. Um im Bereich um den Abhebepunkt des Transportkabels von der Speichertrommel ebenfalls eine knick- bzw. schlaufenfreie Kabelführung zu gewährleisten, kann in diesem Bereich eine Anpreßrolle und/oder ein Kabelführungsteil vorgesehen sein, durch welche das Transportkabel von der Speichertrommel abgehoben wird. - Es versteht sich, daß eine derartige Führung eines Anpreßbandes unabhängig von der Verwendung einer Kabelaufnahmenut vorteilhaft angewendet werden kann. Damit wird ein Tansportkabelantrieb geschaffen, der extrem dünne Transportkabel, wie sie zum Beispiel für die intraarterielle Bestrahlung zur Verhinderung einer postangioplastischen Restenosis erforderlich sind, sicher antreiben kann.

Das Anpreßband kann im Bereich um den Abhebepunkt des Transportkabels von der Speichertrommel gegensinnig zu der Wickelrichtung der Speichertrommel, z. B. mittels Umlenkrollen, um die Speichertrommel geführt werden. Hierdurch können unnötige Überkreuzungen oder Umlenkungen des Anpreßbandes oder auch des Transportkabels vermieden werden und sowohl Anpreßband als auch Transportkabel weitgehend in einer auf der Speichertrommelachse senkrecht stehenden Ebene geführt werden.

Zur Schonung der Strahlenquelle sowie des Transportkabels im Falle von im Transportkanal befindlichen Hindernissen, insbesondere auch aufgrund von Fehlbedienungen, kann das Bestrahlungsgerät einen Anstoßsensor zur Überwachung der auf das Transportkabel ausgeübten Schubkraft umfassen (Anspruch 9). Es versteht sich, daß dieser Anstoßsensor auch unabhängig von den übrigen Merkmalen obengenannten Strahlungsgerätes vorteilhaft Verwendung finden kann.

Zwar kann der Anstoßsensor jedes Mittel zur Überwachung einer auf ein Kabel ausgeübten Schubkraft umfassen, es ist jedoch insbesondere von Vorteil, wenn der Anstoßsensor einen entlang einer Biegung geführten Bereich des Transportkabels sowie Mittel zum Erfassen einer Verlagerung des Transportkabels zur Biegungsaußenseite hin umfaßt (Anspruch 8). Als Biegungsaußenseite wird im Rahmen dieser Offenbarung die von der Biegung nach radial außen gewandte Seite des Kabels verstanden.

Als Verlagerungserfassungsmittel können sämtliche zur Erfassung einer Verlagerung eines Kabels, wie z. B. optische oder elektrische Sensoren, dienen. Besonders vorteilhaft ist jedoch die Verwendung eines mechanischen Schaltkontaktes und/oder eines Näherungsschalters.

Stößt während des Vorschubs der Strahlenquelle diese oder das Transportkabel gegen ein Hindernis, so verlagert sich das Transportkabel im Bereich der Biegung zur Biegungsaußenseite hin. Die Eigenelastizität des Transportkabels kann ausreichend sein, während des Normalbetriebes eine Verlagerung des Transportkabels zur Biegeaußenseite hin zu verhindern, so daß die Verlagerungserfassungmittel während des Normalbetriebes keine Verlagerung erfahren. Es kann jedoch von Vorteil sein, wenn der Anstoßsensor federelastische, einer Verlagerung des Transportkabels zur Biegeaußenseite hin entgegenwirkende Mittel umfaßt. Durch Auswahl der Federkonstante kann die auf das Transportkabel ausübbare Schubkraft eingestellt werden. Derartige federelastische Mittel können z. B. biegeelastische, das Transportkabel führende Röhrchen sein. Ebenso können diese Rohre starr, aber beweglich gegenüber dem übrigen Transportkanal ausgebildet und mit einer Rückhaltefeder, wie z. B. einer an der Biegungsinnenseite angeordnete Zugfeder, verbunden sein. Es versteht sich, daß in ähnlicher Weise auch ein Zugkraftsensor vorgesehen sein kann, der z. B. eine Verlagerung des Transportkabels zur Biegeinnenseite hin überwacht. In einem derartigen Fall können vorteilhaft federelastische Mittel vorgesehen sein, die der Verlagerung des Transportkabels zur Biegeinnenseite entgegenwirken.

Bei Verwendung eines Sensors, der ein zur Auslenkung des gebogenen Transportkanals analoges Ausgangssignal liefert, ist es möglich, über einen Regelverstärker einen Elektromagneten als Rückstellkraft so zu betreiben, daß die Auslenkung beliebig klein gehalten werden kann. Das zur Überwachung der Schubkraft erforderliche Signal liefert in diesem Fall der Regelverstärker.

Es versteht sich, daß die erfindungsgemäße Transportkabelanstoßüberwachung mit einem Anstoßsensor auch bei anderen Bestrahlungsgeräten als denen nach Ansprüchen 1, 3, und/oder 4 vorteilhaft verwendbar sind. Ein Sensor mit analogem Ausgangssignal gestattet ferner eine dynamische Geschwindigkeitsregelung:

Zur Minimierung der Strahlenbelastung ist eine möglichst kurze Ausfahrzeit (< 5 s) erforderlich. Mit zunehmender Ausfahrgeschwindigkeit steigen jedoch die Reibungskräfte zwischen Transportkabel und Führungsschlauch insbesondere bei kleinen Krümmungsradien des Führungsweges.

Mit dem analogen Sensorsignal kann der Sollwert der Ausfahrgeschwindigkeit des Transportkabels entsprechend der Reibungskraft so geregelt werden, daß die auf das Kabel ausgeübte Schubkraft einen bestimmten (vom Kabel abhängigen) Maximalwert nicht überschreitet. So läßt sich bei größtmöglicher Ausfahrgeschwindigkeit eine Überbeanspruchung des Transportkabels und Führungsschlauches verhindern. Eine Unterbrechung der Vorschubbewegung erfolgt nur, wenn bei minimal zulässiger Ausfahrgeschwindigkeit die maximal zulässige Schubkraft überschritten wird. - Auch diese dynamische Geschwindigkeitsregelung ist bei anderen Bestrahlungsgeräten als denen nach Ansprüchen 1, 3 und/oder 4 vorteilhaft verwendbar.

Um Verwechslungen der Strahlenquellen zu vermeiden, kann der Transportbehälter Mittel zum, insbesondere elektronischen, identifizieren der zu ihm gehörenden Strahlenquelle umfassen (Anspruch 9). Es versteht sich, daß diese Identifizierungsmittel unabhängig von den übrigen Merkmalen des Bestrahlungsgerätes vorteilhaft Verwendung finden können. Insbesondere können diese Identifizierungsmittel einen elektronischen Speicher, vorzugsweise ein serielles EEPROM, umfassen (Anspruch 10). Insbesondere ist es möglich, das übrige Bestrahlungsgerät, insbesondere auch automatisch, mit diesen Identifizierungsmitteln zu verbinden, so daß die vorhandene Strahlenquelle von dem Bestrahlungsgerät, insbesondere von dessen Elektronik, erkannt werden kann. Insbesondere können die Identifizierungsmittel auch andere Daten der Strahlenquelle, wie Anfangsaktivität und Anfangsdatum oder ähnliches beinhalten. Ebenso können die Identifizierungmittel Daten des Transportbehälters oder auch des Transportmoduls beinhalten. Diese können insbesondere die Kabellänge des Transportkabels bzw. die Kabelstärke sein. Ebenso können Daten der Speichertrommel bzw. des Transportkabelantriebes, wie z. B. deren Radius oder die Anzahl der Weggeberimpulse pro mm Ausfahrweg, von den Identifizierungsmitteln beinhaltet werden. Auf diese Weise ist trotz einer kostengünstigen Fertigung der Antriebsmechanik, die in der Regel gewisse Fertigungstoleranzen beinhaltet, eine hohe Positioniergenauigkeit möglich, da die mechanischen Daten durch von den Identifizierungsmitteln beinhaltete Daten ausgeglichen bzw. verrechnet werden können.

Um auch höheren Anforderungen an die Abschirmung gerecht zu werden und hierdurch die Exportmöglichkeit zu erhöhen, kann die Abschirmung Baugruppen aus Wolfram aufweisen, da dieses Material keine Eigenstrahlung besitzt.

Die vorgenannten, sowie die beanspruchten und in den Ausführungsbeispielen beschriebenen, erfindungsgemäß zu verwendenden Bauteile unterliegen in ihrer Größe, Formgestaltung, Materialauswahl und technischen Konzeption keinen besonderen Ausnahmebedingungen, so daß die in dem jeweiligen Anwendungsgebiet bekannten Auswahlkriterien uneingeschränkt Anwendung finden können.

Weitere Einzelheiten, Merkmale und Vorteile des Gegenstandes der Erfindung ergeben sich aus der nachfolgenden Beschreibung der zugehörigen Zeichnung, in der - beispielhaft - eine bevorzugte Ausführungsform eines erfindungsgemäßes Bestrahlungsgerät dargestellt ist. In der Zeichnung zeigen
- Fig. 1: ein erfindungsmäßes Bestrahlungsgerät in schematischer Darstellung in Aufsicht;

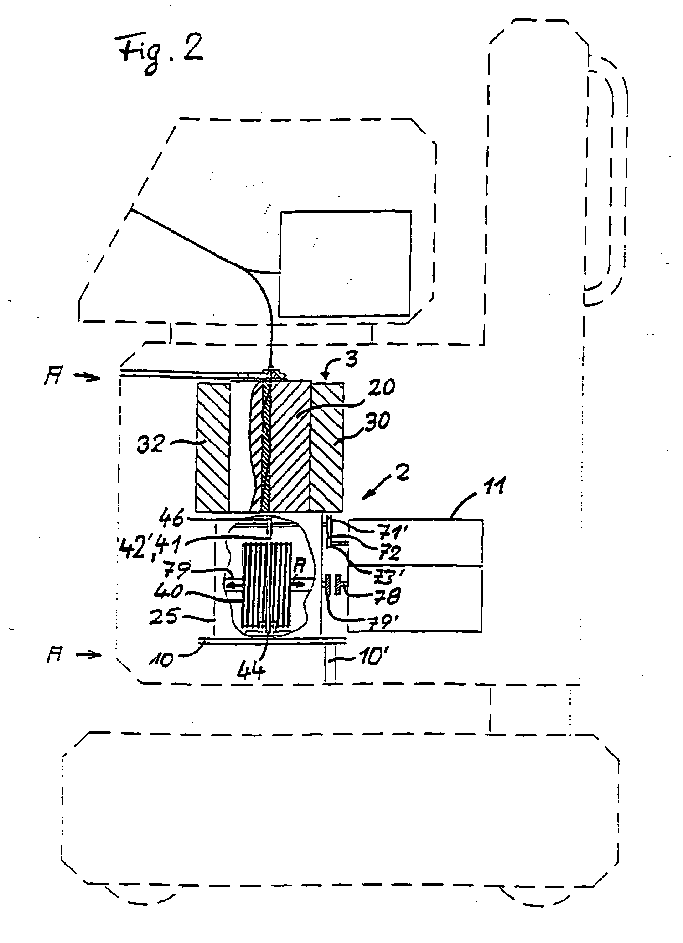

- Fig. 2: dasselbe Bestrahlungsgerät in Seitenansicht, zum Teil aufgebrochen und zum Teil in Vertikalschnittansicht;
- Fig. 3: den Transportbehälter nach Fig. 2 in vergrößerter Seitenansicht, teilweise aufgebrochen;
- Fig. 4: von dem Transportbehälter nach Fig. 1 bis 3 das Transportmodul in Frontansicht (schematisiert und zum Teil vertikal geschnitten) - Ansicht A-A gemäß Fig. 1 und 2;
- Fig. 5: das Transportmodul nach den Fign. 1 und 4 mit Blockiermitteln in schematischer Darstellung;
- Fig. 6: von dem Transportmodul nach den Fign. 1 bis 5 die Speichertrommel im. Schnitt entlang der Linie VI-VI nach Fig. 4;
- Fig. 7: die Befestigung des Transportkabels sowie des Anpreßbandes an der Speichertrommel;
- Fig. 8: eine Klemmvorrichtung für das Anpreßband;
- Fig. 9: die Transportkabelantriebsmittel des Bestrahlungsgerätes nach Fig. 1 in schematischer Darstellung;
- Fig. 10: die Antriebsverbindung zwischen Anpreßband, Antrieb und Bestrahlungsgerätmotor nach Fig. 9 im Schnitt entlang der Linie X-X nach Fig. 9;

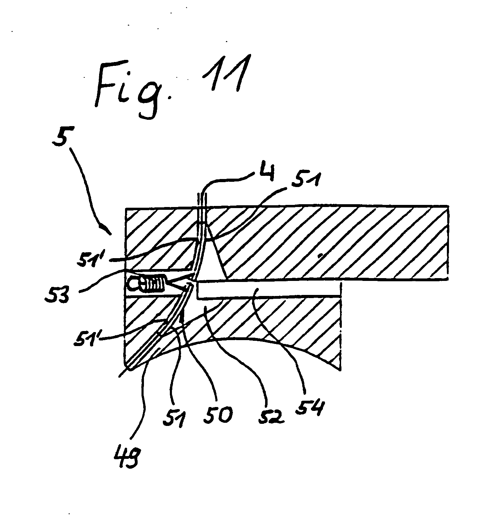

- Fig. 11: einen erfindungsgemäßen Anstoßsensor;
- Fig. 12: einen Transportkanalanschluß zwischen dem Bestrahlungsgerät und einem Transportbehälter;
- Fig. 13: den Transportkanalanschluß nach Fig. 12 in Aufsicht;
- Fig. 14: den Transportkanalanschluß nach den Fign. 12 und 13 im Schnitt entlang der Linie XIV-XIV nach Fig. 13;
- Fig. 15: A bis C ein Transportkabel mit Strahlenquelle in zwei Ausführungsarten in Seitenansicht (Fig. 15A und B) sowie im Querschnitt (Fig. 15c).
- Fig. 16: eine alternative Ausführungsform der Transportkabelführung (Alternative zu Fig. 6) in vergrößerter Ausschnittsdarstellung der Kabelaufnahmenut (Schnitt entlang der Linie XVI - XVI gemäß Fig. 17);
- Fig. 17: von derselben Ausführungsform einer Speichertrommel in Seitenansicht;
- Fig. 18: von derselben Ausführungsform einer Speichertrommel für die Ausführungsform nach Fig. 16 (zum Teil in Axialschnitt - Schnitt entlang der Linie XVIII - XVIII gemäß Fig. 19);
- Fig. 19: von derselben Speichertrommel eine Seitenansicht (Ansicht A gemäß Fig. 18) sowie
- Fig. 20: eine weitere alternative Speichertrommel zu der Ausführungsform nach Fig. 16 in Seitenansicht

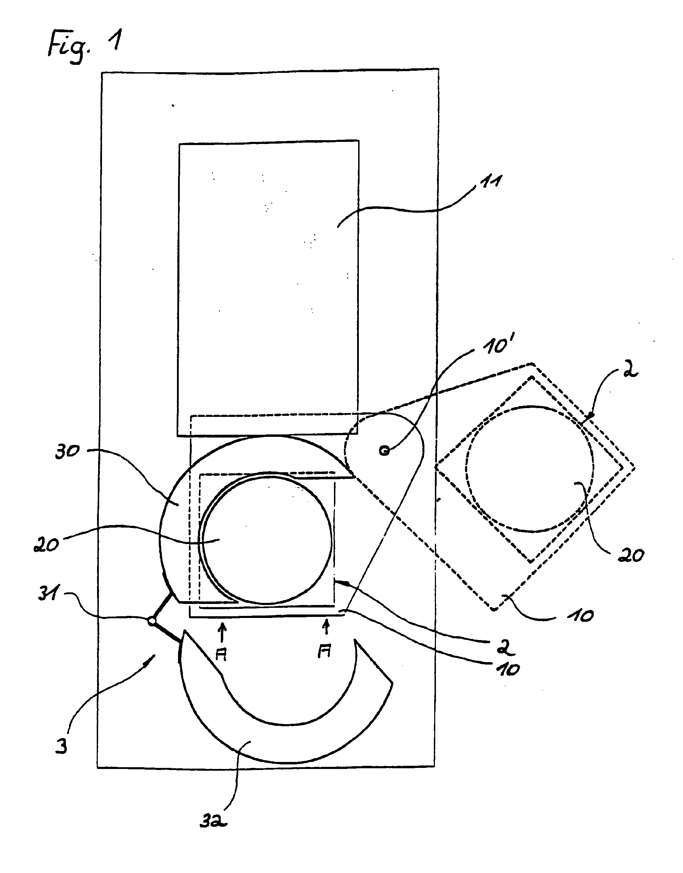

Das Bestrahlungsgerät weist - wie Fig. 1 zeigt - eine Trägerplatte 10 auf, auf welcher ein Transportmodul 2 mit einem Transportbehälter 20 angeordnet ist. Zur Erleichterung des Aus- bzw. Einbaus des Transportmoduls 2 ist die Trägerplatte 10 um eine Achse 10' horizontal schwenkbar (die ausgeschwenkte Position ist in Fig. 1 gestrichelt dargestellt). Im eingeschwenkten Zustand ist der Transportbehälter von einer Zusatzabschirmung 3 umhüllt. Die Zusatzabschirmung 3 weist eine fest mit dem Bestrahlungsgerät verbundene Zusatzabschirmungshälfte 30 sowie eine mittels eines Scharniers 31 aufklappbare Zusatzabschirmungshälfte 32 auf. Die beiden Zusatzabschirmungshälften 30 und 32 sind derart ausgebildet, daß in geöffneten Zustand des Scharniers 31 das Transportmodul 2 mit der Trägerplatte 10 ausgeschwenkt werden kann. Die Schnitte beider Zusatzabschirmunghälften 31 und 32 sind jedoch derart ausgebildet, daß die Schnittebenen zumindest die Lagerposition der Strahlenquelle, und Möglichkeit sogar (wie in Fig. 1 dargestellt und insoweit bevorzugt) den Transportbehälter 20 nicht schneiden. Hierdurch wird ein Austreten von Strahlung entlang der Schnittebenen verhindert.

Das Bestrahlungsgerät weist desweiteren ein Gehäuse 11 für diverse Antriebe, zumindest für ein Transportkabel, sowie diverse Steuereinheiten auf.

Der Transportbehälter 20 umfaßt - vorzugsweise - einen exzentrisch durchbohrten, vorzugsweise aus Wolfram bestehenden Außenzylinder 21, in welchem ein Innenzylinder 22 eingepaßt ist. In die Außenfläche des Innenzylinders 22 ist ein spiralförmiger Kabelkanal eingearbeitet. Ein derartiger Transport- und Strahlenschutzbehälter ist auch ohne die Merkmale der nebengeordneten Ansprüche vorteilhaft verwendbar und einfach herstellbar. Dieser spiralförmige Kabelkanal 23 verläßt den Transportbehälter 20 durch einen auf einer Oberfläche des durch Außenzylinder 21 und Innenzylinder 22 gebildeten Zylinders angeordneten Anschlußkegel 24.

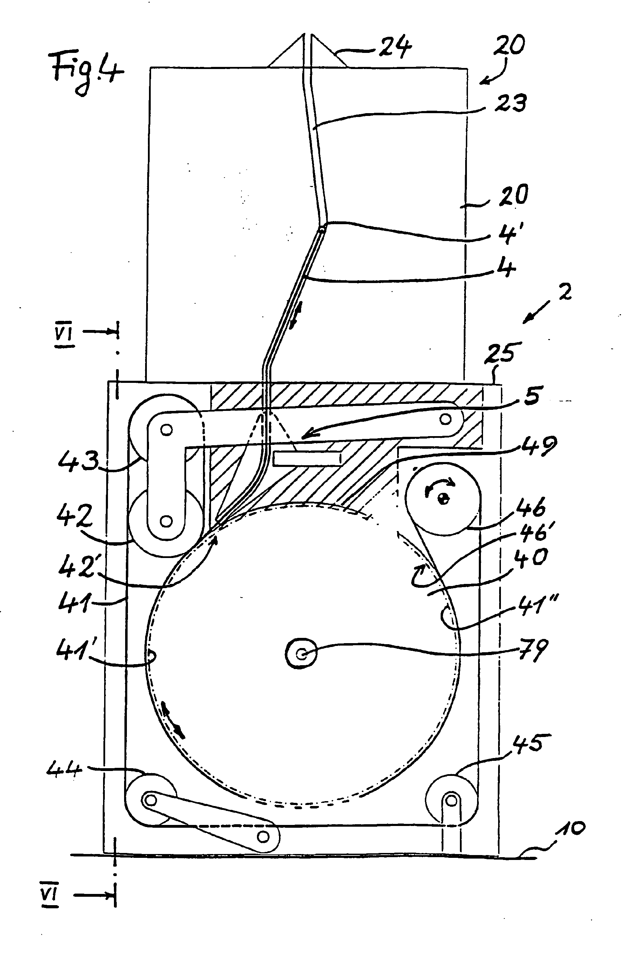

Wie Fig. 4 zeigt, weist das Transportmodul 2 unter seinem Transportbehälter 20 ein Antriebsgehäuse 25 auf, in welchem Baugruppen zum Antrieb eines Transportkabels 4 angeordnet sind. An einem Ende des Transportkabels 4 ist eine Strahlenquelle 4' angebracht. In Fig. 4 ist die Strahlenquelle 4' in ihrer Ruheposition dargestellt.

In dem Antriebsgehäuse 25 ist eine Speichertrommel 40 angeordnet, auf bzw. von welcher das Transportkabel 4 auf- bzw. abgewickelt werden kann. Wie weiter unten näher erläutert, wird das Transportkabel 4 mittels eines Anpreßbandes 41 auf die Speichertrommel 40 gepreßt. Im Bereich des Abhebepunktes 42' des Transportkabels 4 ist eine Anpreßrolle 42 vorgesehen, über welche das Anpreßband 41 von der Speichertrommel 40 abgehoben wird. Das Anpreßband 41 ist mittels Umlenkrollen 43, 44 und 45 sowie einer Antriebsrolle 46 wieder auf die Speichertrommel 40 geführt. Das Anpreßband 41 ist an seiner an der Antriebsrolle 46 anliegenden Seite - wie bevorzugt - als Zahnriemen ausgebildet, so daß ein sicherer Antrieb des Anpreßbandes 41 und somit auch der Speichertrommel 40 und des Transportkabels 4 gewährleistet ist.

Hinter dem Abhebepunkt 42' vor Eintritt in den Transportbehälter 20 weist das Transportkabel 4 einen gebogenen Bereich auf, durch welchen ein weiter unter erläuterter Anstoßsensor 5 gebildet ist.

Im Normalbetrieb wird das Anpreßband 41 mittels der Antriebsrolle 46 angetrieben, so daß sich das Transportkabel 4 von der Speichertrommel 40 abwickelt. Auf diese Weise wird die Strahlenquelle 4' durch den spiralförmigen Kabelkanal 23 und durch den Anschlußkegel 24 aus ihrer Ruheposition in eine Bestrahlungsposition bewegt. Umgekehrt kann die Strahlenquelle 4' durch entsprechenden Antrieb der Antriebsrolle 46 wieder in ihrer Ruheposition zurückbewegt werden.

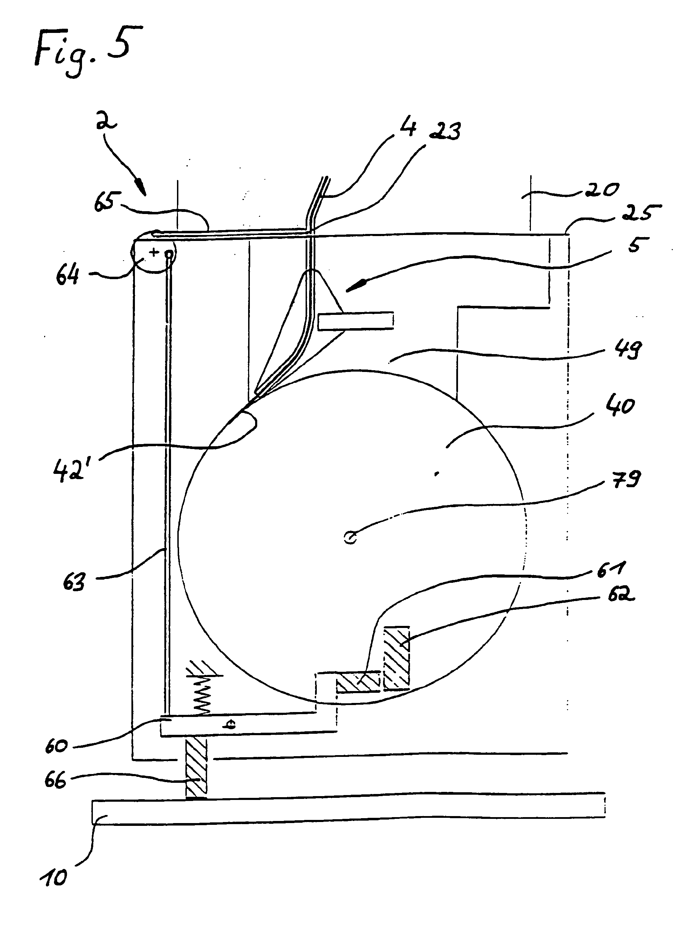

Zur Lagesicherung der Strahlenquelle 4' in ihrer Transportposition während des Transport des Transportmoduls 2 weist diese einen Arretierungshebel 60 (Fig. 5) sowie einen am Antriebsgehäuse 25 angebrachten Anschlag 62 auf, zwischen welchen ein an der Speichertrommel 40 befindlicher Zapfen 61 arretiert werden kann, wenn die Strahlenquelle 4' in ihrer Transportposition ist. Der Arretierungshebel 60 ist des weiteren mit einer Arretierungsstange 63 verbunden, die über ein Getriebe 64 eine weitere Arretierungsstange 65 in den spiralförmigen Kabelkanal 23 pressen kann, so daß das Transportkabel 4 arretiert ist.

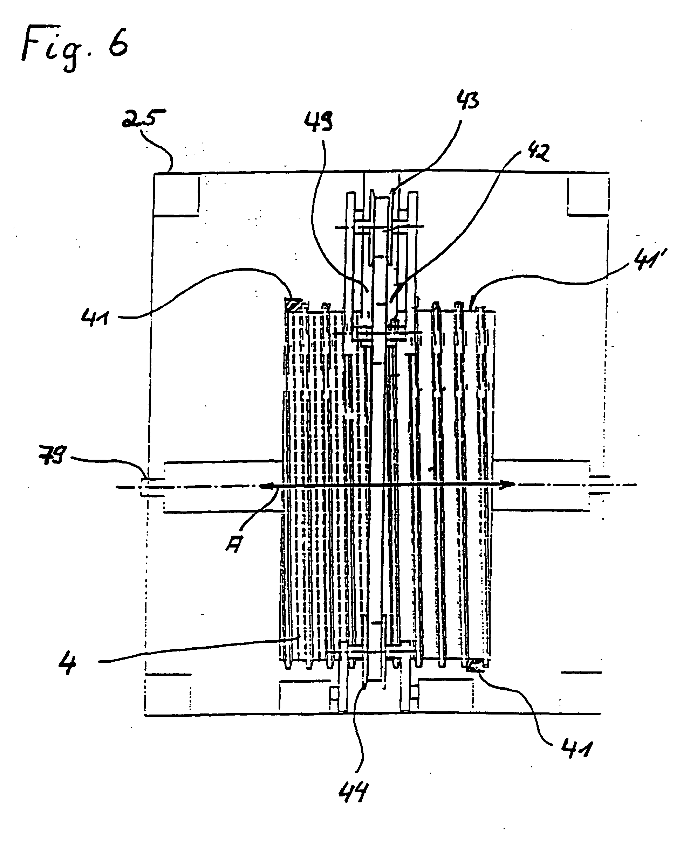

Wird das Transportmodul 2 auf der Trägerplatte 10 gestellt, so kann mittels eines Entriegelungsstiftes 66 der Arretierungshebel 60 verschwenkt werden. Dieses Verschwenken bedingt, daß die Arretierungsstange 65 das Transportkabel 4 freigibt (siehe Fig. 5). Ebenfalls bewirkt dieses Verschwenken des Arretierungshebels 60, daß der Zapfen 61 der Speichertrommel 40 freigegeben wird (dieser freigegebene Zustand ist bei der Darstellung in Fig. 5 noch nicht erreicht). Hat der Arretierungshebel 60 den Zapfen 61 freigegeben, kann die Speichertrommel die Strahlenquelle 4' ausfahren. Die Speichertrommel 40 verschiebt sich - wie im Zusammenhang mit Fig. 6 noch zu erläutern - während ihres Drehens, d. h. während der Bewegung des Transportkabels 4 parallel zur Speichertrommelachse 79. Deshalb hat der Zapfen 61 bezüglich des Anschlags 62 nach einer Trommelumdrehung bereits einen ausreichenden Abstand, um ein freies Drehen der Speichertrommel 40 zu ermöglichen.

Die spiralförmige Anordnung des Anpreßbandes 41 in einer spiralförmigen Kabelaufnahmenut 41' der Speichertrommel 40 ist in Fig. 6 schematisch dargestellt. Das Anpreßband 41 ist in dieser Figur im Schnitt jeweils am Ende der Kabelaufnahmenut 41' dargestellt. Während das Anpreßband 41 praktisch über die gesamte Länge der Kabelaufnahmenut 41' in dieser geführt ist, ist das Transportkabel 4 nur im in Fig. 6 linken Bereich der Speichertrommel 40 auf diese aufgewickelt (gestrichelt dargestellt). Im Bereich des Abhebepunktes 42' des Transportkanals ist auch das Anpreßband 41 mittels der Anpreßrolle 42 sowie der Umlenkrollen 43, 44 und 45 und der Antriebsrolle 46 von der Speichertrommel 40 abgehoben. Wie insbesondere Fig. 6 zeigt, verläuft hierbei das Anpreßband 41 im wesentlichen in einer zur Speichertrommelachse 79 senkrechten Ebene. Ein in die Kabelaufnahmenut 41', 41'' als Führungsschuh gleitend eingreifendes, gehäusefestes Führungsteil 49 zwingt der rotierenden Speichertrommel 40 eine Relativbewegung zum Abhebepunkt 42' auf, so daß sich die Speichertrommel 40 in dem dargestellten und insoweit bevorzugten Ausführungsbeispiel auf ihrer Welle (Achse 79) entlang gleitet.

Die Speichertrommel 40 ist bewegbar - und zwar gleitend verschiebbar - auf der Speichertrommelachse 79 angeordnet, wie durch den Doppelpfeil A angedeutet. Auf diese Weise bleiben der Abhebepunkt 42' sowie die Lage der Anpreßrolle 42, der Umlenkrollen 43, 44, 45 und der Antriebsrolle 46 ortsfest, während das Anpreßband 41 bei 46' in den zum Abhebepunkt 42 benachbarten Teil der Kabelaufnahmenut 41'' in die Anpreßstellung zurückkehrt.

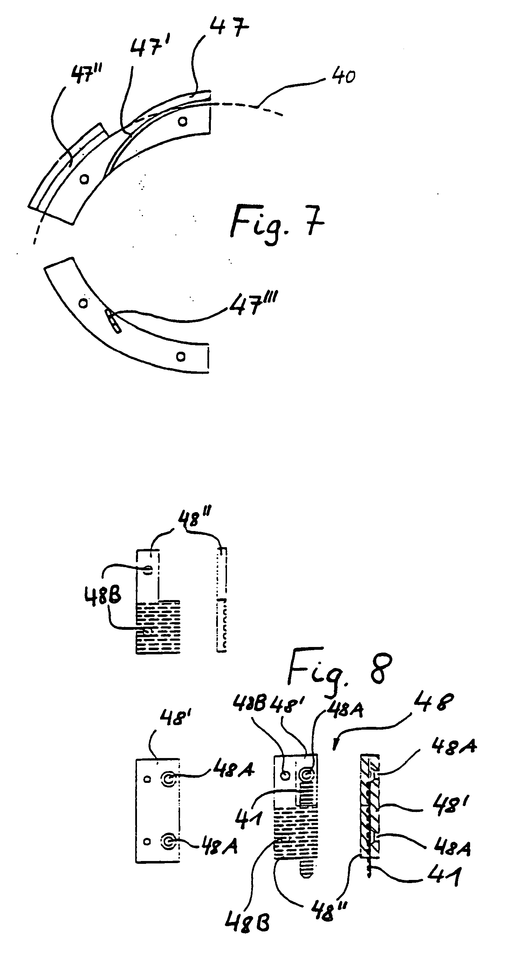

An der einen Seite der Speichertrommel 40 (in Fig. 7 gestrichelt dargestellt) ist eine Halterung 47 angebracht, die einen Klemmkanal 47' für das Transportkabel 4 und eine Anpreßbandhalterung 47'' aufweist. Desweiteren ist an dieser Seite der Speichertrommel eine Transportbandklemme 47''' angebracht, die das Transportband 4 gegenüber der Speichertrommel 40 fixiert. An der anderen Seite der Speichertrommel 40 ist eine Spannvorrichtung 48 für das Anpreßband 41 angebracht, die eine an der Speichertrommel 40 mittels Schrauben 48A befestigte Grundplatte 48' umfaßt, auf die eine Klemmplatte 48'' mit der Verzahnung des Anpreßbandes entsprechenden Ausnehmungen mittels Schrauben 48B aufgeschraubt wird, so daß das Anpreßband 41 mit seiner Verzahnung in die Ausnehmungen eingreift und auf diese Weise in seiner Lage fixiert wird.

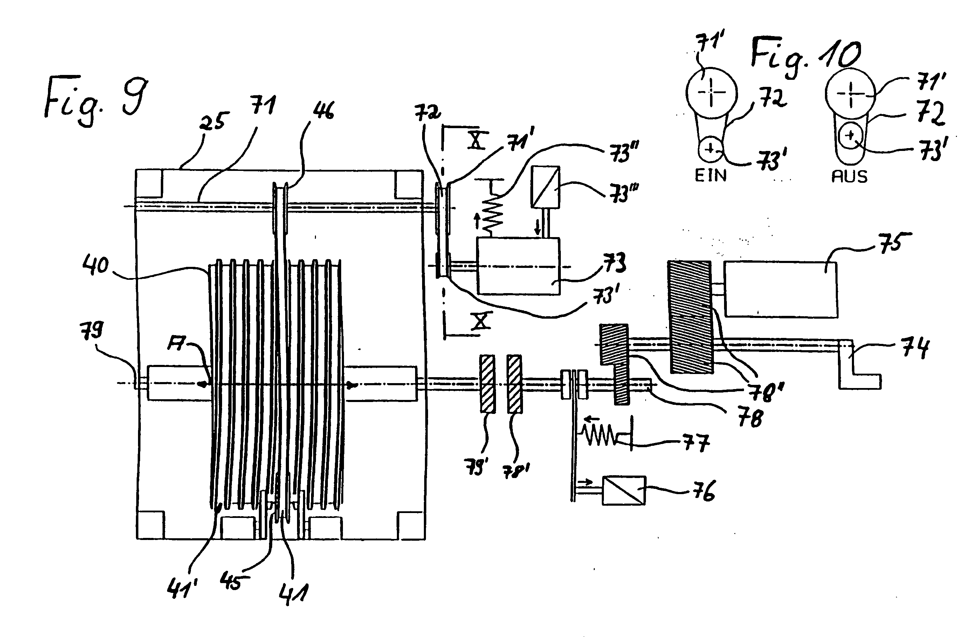

Wie Fig. 9 zeigt, wird die Antriebsrolle 46 und somit auch das Transportkabel 4 im Normalfall über eine mit ihr drehfest verbundene Antriebswelle 71 und eine mit dieser verbundenen Antriebsscheibe 71' angetrieben. Die Antriebsscheibe 71' weist eine Verzahnung auf, in die ein Zahnriemen 72 eingreift. Der Zahnriemen 72 verläuft im Betrieb über eine an einem Motor 73 angebrachten Antriebsscheibe 73'. Der Motor 73 ist über eine Zugfeder 73'' und einen Druckmagneten 73''' zwischen einer EIN-Position und einer AUS-Position derart bewegbar, daß im stromlosen Zustand des Druckmagneten 73''' die Antriebsscheibe 73' außer Eingriff mit dem Zahnriemen 72 ist, während im stromdurchflossenen Zustand der Druckmagnet 73''' die Antriebsscheibe 73' in Eingriff mit dem Zahnriemen 72 bringt (siehe Fig. 10). Hierdurch ist im Notfall, insbesondere bei Stromausfall, die Antriebswelle 71 von dem Motor 73 getrennt und kann frei bewegt werden, um zum Beispiel das Transportkabel 4 wieder auf der Speichertrommel 40 aufzuwickeln. Insbesondere ist auch in der AUS-Position ein Entfernen des Transportmoduls 2 sehr einfach, da dieses dann nicht extra von dem Transportkabelantrieb getrennt werden muß.

Um im Notfall ein Einziehen des Transportkabel 4 und somit auch der Strahlenquelle 4' z. B. in die Transport- bzw. Ruheposition zu ermöglichen, weist das Bestrahlungsgerät einen Notantrieb auf. Der Notantrieb umfaßt eine Kupplungsscheibe 79', die mit der als Drehmomentwelle ausgebildeten Speichertrommelachse 79 verbunden ist. Eine zweite Kupplungsscheibe 78' ist mit einer Achse 78 verbunden, wobei die Achse 78 mit der Kupplungsscheibe 78' mittels einer Druckfeder 77 gegen die erste Kupplungsscheibe 79' gedrückt wird. Durch einen Zugmagneten 76 hingegen werden die Kupplungsscheiben 78' und 79' getrennt, so daß, wenn der Zugmagnet 76 unter Strom steht, der Notantrieb von der Speichertrommelachse getrennt ist. Insbesondere im Falle eines Stromausfalls, jedoch z. B. auch durch manuelle Betätigung, kann der Zugmagnet 76 die Achse 78 freigeben und somit den Notantrieb einkoppeln. Zur Bewegung des Transportkabel weist der Notantrieb eine Handkurbel 74 und einen Notmotor 75 auf, die über Zahnräder 78'' mit der Achse 78 verbunden sind.

Es versteht sich, daß die einzelnen Merkmale vorgenannten Transportkabelantriebs unabhängig von den übrigen Merkmalen des Bestrahlungsgerätes vorteilhaft Verwendung finden können.

Der Anstoßsensor 5 umfaßt einen gebogenen Bereich 50 des Transportkabels 4, welches in diesem gebogenen Bereich 50 in zwei ein wenig voneinander beabstandeten Krümmern 51, vorzugsweise mit einem Biegeradius von 4 cm, geführt ist. Die Krümmer 51 liegen biegungsinnenseitig an Anschlägen 51' an, können sich jedoch in einen an der Biegungsaußenseite vorgesehen Hohlraum 52 verlagern. Durch eine Zugfeder 53 werden die Krümmer 51 in ihrer Position bezüglich der Anschläge 51' gehalten. Stößt nun beim Vorschub das Transportkabel 4 an ein Hindernis, so tendiert es dazu, sich in den Hohlraum 52 bzw. zur Biegungsaußenseite hin zu verlagern. Übersteigt die hierdurch auf die Krümmer 51 ausgeübte Kraft die Zugkraft der Zugfeder 53, so verlagern sich das Transportkabel 4 und die Krümmer 51 in den Hohlraum 52. Diese Verlagerung wird durch einen Schalter 54 erfaßt. Der Schalter 54 sendet ein Signal an die Elektronik des Bestrahlungsgerätes, die ein weiteres Schieben des Transportkabels 4 durch die Speichertrommel 40 verhindert.

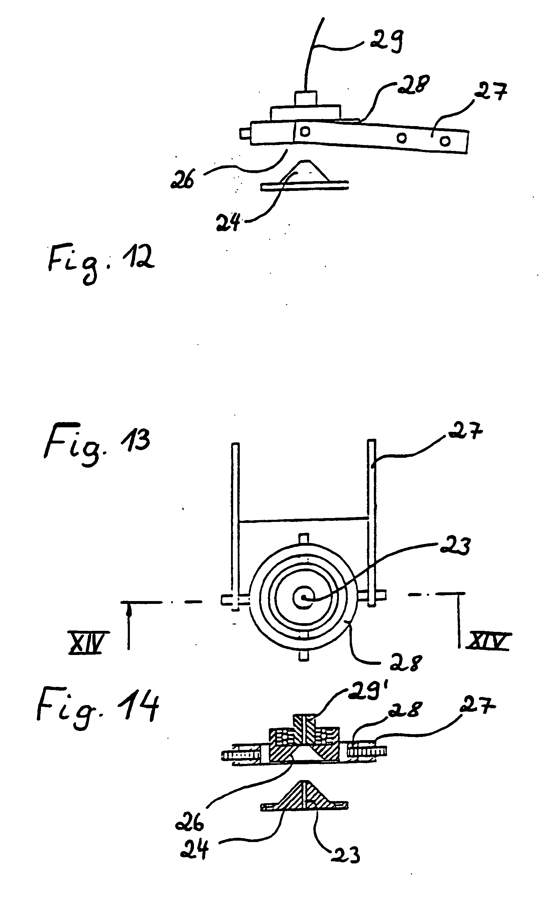

Um eine funktionssichere und schnell zu verbindende bzw. lösende Verbindung zwischen dem spiralförmigen Kabelkanal 23 des Transportbehälters 20 einem Verbindungsschlauch 29 zu gewährleisten, weist dieser den Anschlußkegel 24 auf, durch welchen der spiralförmige Kabelkanal 23 austritt. Desweiteren weist hierzu das Bestrahlungsgerät einen Anschlußkonus 26 auf, welcher auf den Anschlußkegel 24 gesetzt werden kann (siehe Fign. 12 bis 14). Zur Erhöhung des Bedienungskomforts und der Betriebssicherheit ist der Anschlußkonus 26 mittels eines beweglichen Armes 27 mit dem übrigen Bestrahlungsgerät verbunden. Um ein sauberes Aufsetzen des Anschlußkonusses 26 auf dem Anschlußkegel 24 sicher zu stellen, ist der Anschlußkonus 26 mit dem Arm 27 über ein Kardangelenk 28 verbunden.

Mittig weist der Anschlußkonus 26 einen Kabelkanal 29' auf, der in einen Verbindungsschlauch 29 übergeht. Der Kabelkanal 29' ist in einem Anschlußstück 26' angeordnet, welches über ein Kugellager 26'' mit dem übrigen Anschlußkonus 26 verbunden ist, um eine Drehbarkeit des Verbindungsschlauches 29 gegenüber dem Anschlußkonus 26 zu gewährleisten. Hierdurch können die auf den Verbindungsschlauch 29 und auf die Verbindung zwischen Anschlußkonus 26 und Anschlußkegel 24 wirkenden Kräfte verringert werden.

Es versteht sich, daß durch eine derartige Verbindung eines Transportbehälters 20 mit einem Verbindungsschlauch 29 auch unabhängig von den übrigen Merkmalen des Bestrahlungsgerätes eine funktionssichere und einfach zu bedienende Verbindung eines Transportbehälters 20 mit einem Verbindungsschlauch 29 verwirklicht werden kann.

Mit der erfindungsgemäßen Speichertrommel mit spiralförmigem Anpreßband können extrem dünne Transportkabel sowie faserförmige, kurzreichweitige Strahlenquellen, insbesondere Beta-Strahler, Verwendung finden, wie sie - grundsätzlich - bereits aus der EP-A1-0 633 041 bekannt sind. Figur 14A zeigt die Spitze eines derartigen Transportkabels 4, welche als, gegebenenfalls auch J-förmig gebogene Spitze, zum Beispiel zum Führen eines Katheters um enge Kurven, dienen kann. Eine Verklebung oder Verpreßung oder Verschweißung 4'' schafft die Verbindung der faserförmigen Strahlenquellen 4' mit dem Antriebskabel 4. Figur 15B zeigt eine entsprechende Strahlenquelle ohne Führungspitze. Figur 15C einen Querschnitt.

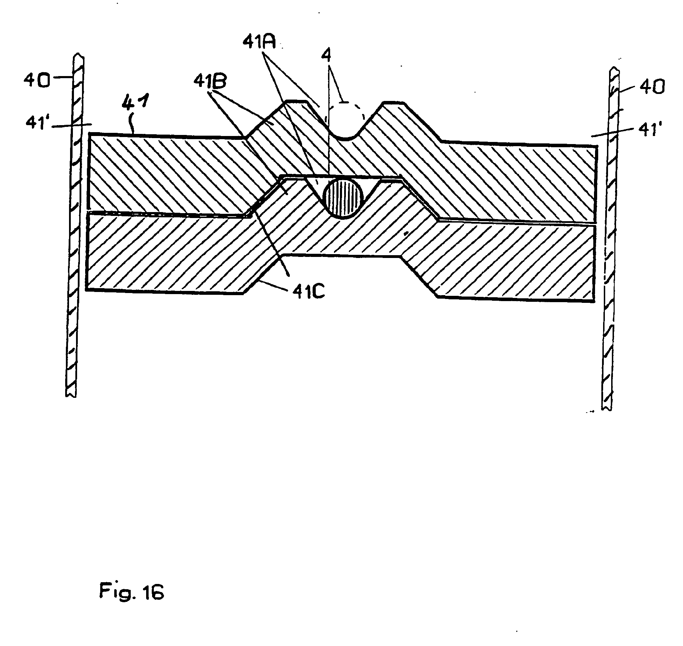

Bei den Ausführungsformen nach Fign. 16 bis 20 erfolgt die spiralförmige Aufwicklung des Transportkabels 4 und des jeweils nur einen Gang des Transportkabels abdeckenden Anpreßbandes 41 in einer bezüglich der Speichertrommel 40 radialen Richtung, so daß das Transportkabel 4 zwischen dem es von radial außen her abdeckenden und anpreßenden Teil eines un-unterbrochenden Anpreßbandes und der radialen Außenseite des zu jeweils zur Achse hin nächstgelegenen Ganges des Anpreßbandes 41 eng geführt wird. Zu diesem Zweck weist das Anpreßband 41 die aus Fig. 16 ersichtliche komplementäre Oberflächenkonfiguration auf seinen beiden radialen Außenflächen auf, insbesondere nach Art der in Fig. 16 dargestellten und insoweit bevorzugten Nut/Feder - Anordnung mit einer Transportkabelführungsnut 41A auf der Seite der "Feder" 41 B.

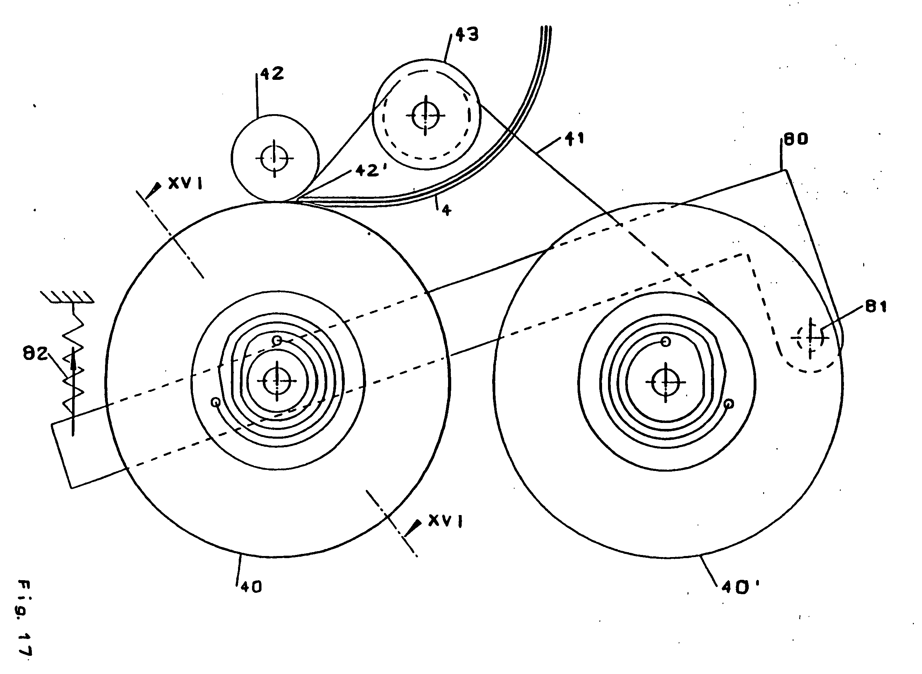

Diese Transportkabel- und Anpreßbandführung kann in verschiedenster Form realisiert werden. So kann der das Transportkabel noch nicht bzw. nicht mehr abdeckende Teil des Anpreßbandes auf eine Speichertrommel 40' aufwickelbar bzw. von ihr abwickelbar sein. Diese Ausführungsformen sind in Fign. 17 bis 20 dargestellt.

Um die unterschiedlichen Radien zu berücksichtigen, wie an der Austrittsstelle 42' des Transportkabels von der Speichertrommel 40 durch die "radiale" Abwicklungsart zwangsläufig entstehen, ist bei der Ausführungsform nach Fig. 17 vorgesehen, daß beide Speichertrommeln 40 und 40' auf einer gemeinsamen Schwinge 80 auf voneinander beabstandeten Achsen drehbar gelagert sind. Die Austrittsstelle 42' bleibt gerätefest während die Speichertrommel 40 und 40' um den Drehpunkt 81 der Schwinge 80 unter dem Zug einer Feder 82 der Radienzu- bzw. -abnahme des Transportkabels 4 und des Anpreßbandes 41 entsprechend nachgeführt wird.

Das Anpreßband muß unter allen Betriebsbedingungen das Transportkabel sicher führen. Dazu sollte das Anpreßband unter einer bestimmten Zugspannung stehen. Diese Zugspannung kann z. B. durch gegeneinander wirkende Spiralfedern in den Speicherspulen 40 und 40' bewirkt werden, wobei die Federkraft der Rückholspule in jedem Fall größer als die der Vortriebsspule sein sollte (Fig. 17).

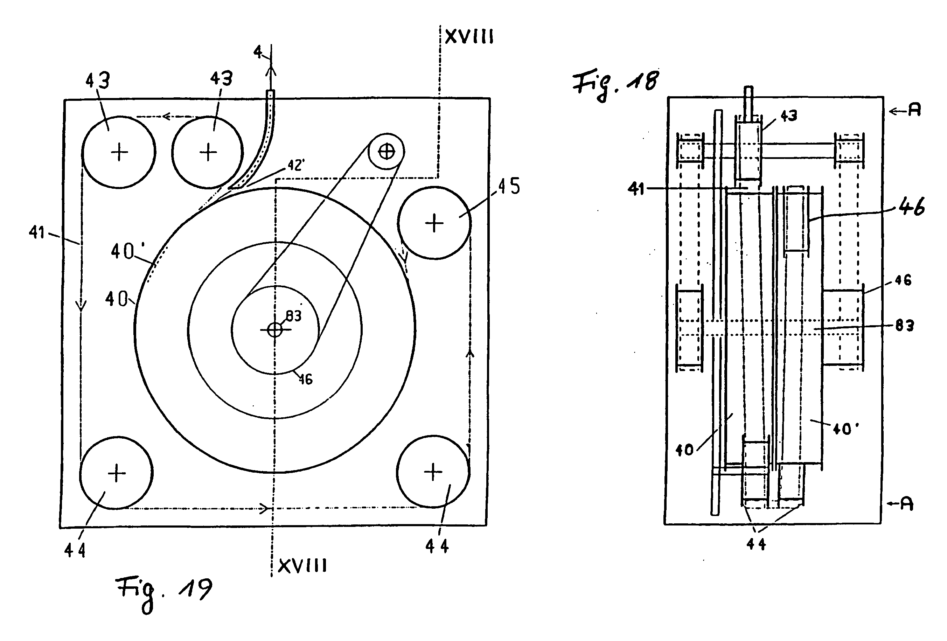

Eine andere Möglichkeit besteht darin, die Zugspannung durch vorgespannte, auf das Anpreßband wirkende Rollen zu erzeugen (Fign. 18 und 19). Dies ist jedoch nur möglich, wenn die beiden Spulen 40 und 40' miteinander mechanisch gekoppelt sind. Zu diesem Zweck sind die Spulen 40 und 40' bei der Ausführungsform nach Fign. 18 und 19 drehbar auf einer gemeinsamen Antriebswelle 23 befestigt. Deshalb ist es erforderlich, die Umlenkrollen 43, 44 und 45 des Anpreßbandes 41 so anzuordnen, daß das Anpreßband zwischen den in axialer Richtung beabstandeten Speichertrommel 40 und 40' hin- bzw. herführbar ist - etwa wie bei einer Ketten-Gangschaltung eines Fahrrades, wobei allerdings nur Antriebsräder und keine Abtriebsräder vorgesehen sind.

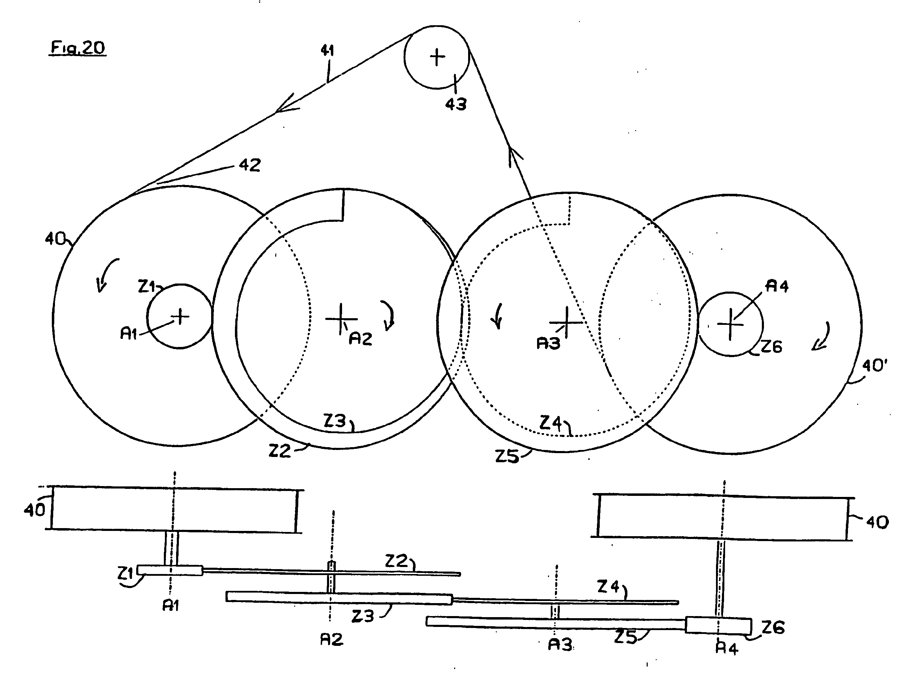

Wegen des veränderlichen Wickeldurchmessers auf den Speichertrommeln 40 und 40' muß sich beim Auf- oder Abwickeln des Anpreßbandes die relative Drehzahl der Speichertrommeln 40 und 40' ändern, wenn der Führungsweg des Anpreßbandes 41 zwischen den beiden Speichertrommeln konstant sein soll. Eine dem Wickeldurchmesser entsprechende relative Drehzahl läßt sich durch ein Getriebe mit zwei spiralförmig geformten Zahnrädern Z3 und Z4 gemäß Fig. 20 realisieren. Wenn das Anpreßband 41 z. B. in zehn Lagen von der Speichertrommel 40 auf die Speichertrommel 40' umgewickelt wird, kann durch eine Untersetzung von 10:1 mittels der Zahnräder Z1:Z2 bzw. Z6:Z5 der gesamte Drehweg der Speichertrommel 40 auf eine einzige Umdrehung der beiden spiralförmigen Zahnräder Z3 und Z4 transformiert werden. Das variable Übersetzungsverhältnis dieser beiden Zahnräder ist so gewählt, daß die Ab- und Aufwickelgeschwindigkeit beider Speichertrommeln 40 und 40' unabhängig vom Wickeldurchmesser ist.

### Bezugszeichenliste

- 10: Trägerplatte
- 10': Achse
- 11: Antriebsgehäuse

- 2: Transportmodul
- 20: Transportbehälter
- 21: Außenzylinder
- 22: Innenzylinder
- 23: spiralförmiger Kabelkanal
- 24: Anschlußkegel
- 25: Antriebsgehäuse
- 26: Anschlußkonus
- 27: Arm
- 28: Kardangelenk
- 29: Verbindungsschlauch
- 29': Kabelkanal des Anschlußkonusses

- 3: Zusatzabschirmung
- 30: Zusatzabschirmungshälfte, fest
- 31: Schanier
- 32: Zusatzabschirmungshälfte, aufklappbar

- 4: Transportkabel
- 4': Strahlenquelel
- 4": Verklebung, Verpreßung, Verschweißung
- 40: Speichertrommel
- 40': Speichertrommel
- 41: Anpreßband
- 41A: Transportkabelführungsnut
- 41': Kabelaufnahmenut
- 41": Kabelaufnahmenut
- 41B: Feder
- 41C: Nut
- 42: Anpreßrolle
- 42': Austrittsstelle
- 43: Umlenkrolle
- 44: Umlenkrolle
- 45: Umlenkrolle
- 46: Antriebsrolle
- 47: Halterung
- 47': Klemmkanal
- 47'': Anpreßbandhalterung
- 48: Spannvorrichtung
- 48': Grundplatte
- 48": Klemmplatte
- 48A: Schrauben
- 48B: Schrauben
- 49: Führungsteil

- 5: Anstoßsensor
- 50: gebogener Bereich
- 51: Krümmer
- 51': Anschlag
- 52: Hohlraum
- 53: Zugfeder
- 54: Schalter

- 60: Arretierungshebel
- 61: Zapfen
- 62: Anschlag
- 63: Arretierungsstange
- 64: Getriebe
- 65: Arretierungsstange
- 66: Entriegelungsstift

- 71: Antriebswelle
- 71': Antriebsscheibe
- 72: Zahnriemen
- 73: Motor
- 73': Antriebsscheibe
- 73": Zugfeder
- 73''': Druckmagnet
- 74: Handkurbel
- 75: Notmotor
- 76: Zugmagnet
- 77: Druckfeder
- 78: Achse
- 78': Kupplungsscheibe
- 78'': Zahnrad
- 79: Speichertrommelachse
- 79': Kupplungsscheibe
- 80: Schwinge
- 81: Drehpunkt
- 82: Feder
- 83: Antriebswelle

## Patentansprüche

1. Bestrahlungsgerät mit zumindest einer mittels eines Transportkabels zwischen zumindest einer Ruheposition und zumindest einer Bestrahlungsposition bewegbaren, auswechselbaren Strahlenquelle und mit einem Transportbehälter für den Fern-Transport auszuwechselnder Strahlungsquellen, wobei
sich die Ruheposition innerhalb des Transportbehälters (20) (Fern-Transportbehälter) befindet und der Transportbehälter Bestandteil des Bestrahlungsgerätes ist und zum Integrieren mit dem verbleibenden Teil des Bestrahlungsgerät gestaltet ist, und wobei der Integrierbarkeit dienende Schnittstellenmittel an dem Transportbehälter und an dem übrigen Teil des Bestrahlungsgerätes vorgesehen sind
**dadurch gekennzeichnet, dass**
der Transportbehälter (20) eine erste Abschirmung aufweist und der verbleibende Teil des Bestrahlungsgeräts eine zweite Abschirmung aufweist, wobei die erste und zweite Abschirmung zusammen eine Strahlenschutzfunktion bereitstellen.

2. Bestrahlungsgerät nach Anspruch 1, **dadurch gekennzeichnet, daß** die erste Abschirmung (21, 22) des Transportbehälters (20) einer für Strahlenquellentransportbehälter geltende Bestimmung entspricht, und dass die zweite Abschirmung (3, 30, 32) des Bestrahlungsgerätes zumindest Teile des Transportbehälters derart umgibt, daß das Bestrahlungsgerät bei in der Ruheposition befindlicher Strahlenquelle einer für Strahlenquellenlagerbehälter geltenden Bestimmung entspricht.

3. Bestrahlungsgerät nach dem Oberbegriff von Anspruch 1,insbesondere nach Anspruch 1 oder 2, **gekennzeichnet durch** ein Transportmodul (2), welches zumindest den Transportbehälter (20) und ein Transportkabelspeicher, auf welche bzw. von welcher das Transportkabel (4) aufbzw. abwickelbar ist, umfaßt.

4. Bestrahlungsgerät nach dem Oberbegriff von Anspruch 1 oder 3, insbesondere nach einem der Ansprüche 1 bis 3, bei der das Transportkabel (4) auf bzw. von einer Speichertrommel (40) mit einer Kabelaufnahmenut (41') aufbzw. abgewickelt wird.

5. Bestrahlungsgerät nach Anspruch 4, **dadurch gekennzeichnet, daß** ein Führungsteil (49) im Bereich der Transportkabelaustrittsstelle (42') vorgesehen ist, das zumindest aber das Transportkabel (4) aus der Kabelaufnahmenut (41') heraus- bzw. in sie einführt.

6. Bestrahlungsgerät nach einem der Ansprüche 1 bis 5, **gekennzeichnet durch** Mittel (60, 61, 62, 63, 64, 65, 66) zum automatischen Blockieren der Strahlenquelle (4') bzw. des Transportkabels (4) in einer Transportposition beim Transport des Transportbehälters (20).

7. Bestrahlungsgerät nach einem der Ansprüche 1 bis 6, **gekennzeichnet durch** einen Anstoβsensor (5) zur Überwachung der auf das Transportkabel (4) ausgeübten Schubkraft.

8. Bestrahlungsgerät nach Anspruch 7, **dadurch gekennzeichnet, daß** der Anstoßsensor (5) einen entlang einer Biegung geführten Bereich (50) des Transportkabels (4) sowie Mittel (54) zum Erfassen einer Verlagerung des Transportkabels (4) zur Biegungsaußenseite hin umfaßt.

9. Bestrahlungsgerät nach Anspruch 7 oder 8, **dadurch gekennzeichnet, daß** ein zumindest gekrümmter Transportkabelbereich (Krümmer 51) zwei freie Enden an einer Unterbrechungsstelle aufweist.

10. Bestrahlungsgerät nach Anspruch 9, **gekennzeichnet durch** zumindest eine auf zumindest eines der freien Enden zum Einstellen einer Auslenkkraft einwirkenden Rückstellfeder (53).

11. Bestrahlungsgerät nach einem der Ansprüche 4 bis 5, ferner aufweisend elektronische Mittel zum Identifizieren der Strahlenquelle (4'), Anfangsaktivität, -datum, und/oder anderer Kenngrößen der Speichertrommel aufweist.

12. Bestrahlungsgerät nach Anspruch 11, **dadurch gekennzeichnet, daß** die elektronischen Mittel einen elektronischen Speicher umfassen.

13. Bestrahlungsgerät nach einem der Ansprüche 1 bis 12, **dadurch gekennzeichnet, daß** der Transportbehälter 20 eine durchbohrte Abschirmung aus Wolfram umfaßt.

14. Bestrahlungsgerät nach einem der Ansprüche 1 bis 13, **dadurch gekennzeichnet, daß** der Transportbehälter (20) aus
einem Außenzylinder (21),
einem in eine Bohrung im Außenzylinder (21) eingepaßten Innenzylinder (22) und
einem an der Berührungsfläche zwischen dem Innenzylinder (22) und der Bohrung im Außenzylinder (21) eingearbeiteten spiralförmigen Kabelkanal (23),
besteht, oder vorgenannte Teile aufweist.

15. Bestrahlungsgerät nach einem der Ansprüche 1 bis 14, **gekennzeichnet durch** eine dynamische Geschwindigkeitsregelung des Transportkabels (4) im Zusammenwirken mit einem Fahrwiderstand - Erfassungssensor.

16. Bestrahlungsgrät nach einem der Ansprüche 1 bis 15, **gekennzeichnet, durch** entlang des Antriebskabels (4) sich erstreckende faserförmige Strahlenquellen (4').

17. Verfahren zur intraarteriellen Bestrahlung zur Verhinderung einer postangioplastischen oder anderweitig zu befürchtenden Restenosis **gekennzeichnet durch** die Verwendung eines Bestrahlungsgerätes nach einem der Ansprüche 1 bis 16.

## Claims

1. An irradiation unit having at least one exchangeable radiation source movable by means of a transport cable between at least one inoperative position and at least one irradiation position and having a transport container for the long-distance transport of radiation sources to be exchanged, wherein
the inoperative position is located within the transport container (20) (long-distance transport container) and the transport container is a component of the irradiation unit and is designed for integration with the remaining part of the irradiation unit, and wherein interface means serving for the integration are provided on the transport container and on the remaining part of the irradiation unit, **characterised in that** the transport container (20) comprises a first shield and the remaining part of the irradiation unit comprises a second shield, the first and second shields together providing a radiation protection function.

2. An irradiation unit according to claim 1, **characterised in that** the first shield (21, 22) of the transport container (20) complies with a regulation applicable to radiation source transport containers, and **in that** the second shield (3, 30, 32) of the irradiation unit surrounds at least parts of the transport container in such a way that in the inoperative position of the radiation source, the irradiation unit complies with a regulation applicable to radiation source storage containers.

3. An irradiation unit according to the preamble of claim 1, in particular according to claim 1 or 2, **characterised by** a transport module (2), which comprises at least the transport container (20) and a transport cable storage device, on which or from which the transport cable (4) can be wound or unwound respectively.

4. An irradiation unit according to the preamble of claim 1 or 3, in particular according to any one of claims 1 to 3, in which the transport cable (4) is wound onto or unwound from a storage drum (40) having a cable locating groove (41').

5. An irradiation unit according to claim 4, **characterised in that** in the region of the transport cable exit point (42') there is provided a guide member (59), which guides at least the transport cable (4) out of and into the cable locating groove (41').

6. An irradiation unit according to any one of claims 1 to 5, **characterised by** means (60, 61, 62, 63, 64, 65, 66) for automatically blocking the radiation source (4') and the transport cable (4) in a transport position during transport of the transport container (20).

7. An irradiation unit according to any one of claims 1 to 6, **characterised by** an impact sensor (5) for monitoring the shear force exerted on the transport cable (4).

8. An irradiation unit according to claim 7, **characterised in that** the impact sensor (5) comprises a region (50) of the transport cable (4) guided along a bend as well as means (54) for detecting a displacement of the transport cable (4) towards the outside of the bend.

9. An irradiation unit according to claim 7 or 8, **characterised in that** an at least curved transport cable region (bend 51) has two free ends at a break point.

10. An irradiation unit according to claim 9, **characterised by** at least one restoring spring (53) acting on at least one of the free ends to adjust a deflection force.

11. An irradiation unit according to any one of claims 4 to 5, further comprising electronic means for identifying the radiation source (4'), initial activity, starting date and/or other characteristics of the storage drum.

12. An irradiation unit according to claim 11, **characterised in that** the electronic means comprise an electronic memory.

13. An irradiation unit according to any one of claims 1 to 12, **characterised in that** the transport container (20) comprises a perforated tungsten shield.

14. An irradiation unit according to any one of claims 1 to 13, **characterised in that** the transport container (20) consists of
an outer cylinder (21),
an inner cylinder (22) fitted into a bore in the outer cylinder (21) and
a helical cable channel (23) formed at the interface between the inner cylinder (22) and the bore in the outer cylinder (21), or comprises the aforementioned parts.

15. An irradiation unit according to any one of claims 1 to 14, **characterised by** a dynamic speed control of the transport cable (4) co-operating with a tractive resistance detection sensor.

16. An irradiation unit according to any one of claims 1 to 15, **characterised by** filamentous radiation sources (4') extending along the drive cable (4).

17. A method for intra-arterial irradiation for prevention of a post-angioplasty restenosis or other feared restenosis, **characterised by** the use of an irradiation unit according to any one of claims 1 to 16.

## Revendications

1. Appareil d'irradiation, comprenant au moins une source de rayonnement renouvelable et pouvant être déplacée au moyen d'un câble de transport entre au moins une position de repos et au moins une position d'irradiation, et un récipient de transport pour le transport à distance de sources de rayonnement renouvelables,
la position de repos se trouvant à l'intérieur du récipient de transport (20) (récipient de transport à distance) et le récipient de transport étant lui-même composant de l'appareil d'irradiation, conçu pour être intégré à la partie restante de l'appareil d'irradiation, et des moyens d'interface servant à l'intégration étant prévus sur le récipient de transport et sur la partie restante de l'appareil d'irradiation,
**caractérisé en ce que**
le récipient de transport (20) comporte un premier blindage et la partie restante de l'appareil d'irradiation un deuxième blindage, le premier et le deuxième blindages ayant ensemble une fonction de protection contre les radiations.

2. Appareil d'irradiation selon la revendication 1, **caractérisé en ce que** le premier blindage (21, 22) du récipient de transport (20) satisfait à une disposition applicable à des récipients de transport des sources de rayonnement, et **en ce que** le deuxième blindage (3, 30, 32) de l'appareil d'irradiation entoure au moins des parties du récipient de transport, de telle manière qu'en cas de source de rayonnement se trouvant en position de repos, l'appareil d'irradiation satisfait à une disposition applicable à des récipients de stockage des sources de rayonnement.

3. Appareil d'irradiation selon le préambule de la revendication 1, en particulier selon la revendication 1 ou la revendication 2,
**caractérisé par** un module de transport (2), lequel comprend au moins le récipient de transport (20) et un magasin à câble de transport, dans lequel ou duquel le câble de transport (4) est enroulable ou déroulable.

4. Appareil d'irradiation selon le préambule de la revendication 1 ou de la revendication 3, en particulier selon l'une des revendications 1 à 3, où le câble de transport (4) est enroulé sur un tambour d'alimentation (40) ou déroulé dudit tambour au moyen d'une rainure de logement du câble (41').

5. Appareil d'irradiation selon la revendication 4, **caractérisé en ce qu'**une pièce de guidage (49) est prévue au niveau du point de sortie du câble de transport (42'), laquelle déloge toutefois au moins le câble de transport (4) de la rainure de logement du câble (41'), ou l'engage dans celle-ci.

6. Appareil d'irradiation selon l'une des revendications 1 à 5, **caractérisé par** des moyens (60, 61, 62, 63, 64, 65, 66) pour le blocage automatique dans une position de transport de la source de rayonnement (4') ou du câble de transport (4) lors du transport du récipient de transport (20).

7. Appareil d'irradiation selon l'une des revendications 1 à 6, **caractérisé par** un capteur d'impulsion (5) destiné à surveiller la force de poussée exercée sur le câble de transport (4).

8. Appareil d'irradiation selon la revendication 7, **caractérisé en ce que** le capteur d'impulsion (5) présente une zone (50) du câble de transport (4) guidée le long d'un arc ainsi que des moyens (54) de saisie d'un décalage du câble de transport (4) vers le côté extérieur de l'arc.

9. Appareil d'irradiation selon la revendication 7 ou la revendication 8, **caractérisé en ce qu'**une zone de câble de transport au moins courbée (courbure 51) comporte deux extrémités libres à un emplacement d'interruption.

10. Appareil d'irradiation selon la revendication 9, **caractérisé par** un ressort de rappel (53) agissant sur au moins une des extrémités libres pour le réglage d'une force de déviation.

11. Appareil d'irradiation selon l'une des revendications 4 ou 5, comprenant en outre des moyens électroniques pour l'identification de la source de rayonnement (4'), l'activité initiale, la date initiale, et/ou d'autres grandeurs caractéristiques du tambour d'alimentation.

12. Appareil d'irradiation selon la revendication 11, **caractérisé en ce que** les moyens d'identification comprennent une mémoire électronique.

13. Appareil d'irradiation selon l'une des revendications 1 à 12, **caractérisé en ce que** le récipient de transport 20 comporte un blindage en wolfram, pourvu d'alésages traversants.

14. Appareil d'irradiation selon l'une des revendications 1 à 13, **caractérisé en ce que** le récipient de transport (20) se compose
d'un cylindre extérieur (21),
d'un cylindre intérieur (22) ajusté dans un alésage du cylindre extérieur (21) et
d'une canalisation de câble (23) hélicoïdale ménagée sur la surface de contact entre le cylindre intérieur (22) et l'alésage du cylindre extérieur (21), ou comporte des éléments susmentionnés.

15. Appareil d'irradiation selon l'une des revendications 1 à 14, **caractérisé par** une régulation dynamique de vitesse du câble de transport (4) en coopération avec un capteur de saisie de résistance de roulement.

16. Appareil d'irradiation selon l'une des revendications 1 à 15, **caractérisé par** des sources de rayonnement (4') en forme de fibres qui s'étendent le long du câble d'entraînement (4).

17. Procédé d'irradiation intra-artérielle, pour empêcher une resténose post-angioplastique ou à redouter pour une autre raison, **caractérisé par** l'utilisation d'un appareil d'irradiation selon l'une des revendications 1 à 16.
